# EUROPÄISCHE PATENTSCHRIFT

(11) **EP 3 366 223 B1**
(45) Veröffentlichungstag und Bekanntmachung des Hinweises auf die Patenterteilung: **01.05.2019**
(21) Anmeldenummer: 17157882.6
(22) Anmeldetag: 24.02.2017
(51) Int. Cl.: A61B 10/00

(54) **LABORBECHER**
LABORATORY CUP
BÉCHER DE LABORATOIRE

(43) Veröffentlichungstag der Anmeldung: 29.08.2018
(73) Patentinhaber: Protzek Gesellschaft für Biomedizinische Technik GmbH, 4133 Prattein (CH)
(72) Erfinder: Protzek, Christoph, 79539 Lörrach (DE)
(74) Vertreter: Patentanwälte Dörner & Kötter PartG mbB

(56) Entgegenhaltungen:
- WO-A1-2015/145154
- WO-A1-2017/013446
- GB-A- 2 529 713
- US-A- 3 746 240

## Beschreibung

Die Erfindung betrifft einen Laborbecher, insbesondere Urinbecher, nach dem Patentanspruch 1.

Täglich werden in Kliniken, Krankenhäusern, Laborinstituten aber auch im mobilen Einsatz Proben, insbesondere Urinproben zum Nachweis z.B. von Drogen, Medikamenten oder Antikörpern als Zeichen für bestimmte Erkrankungen genommen, um diese einer nachfolgenden Laboruntersuchung zuzuführen. Hierzu kommen regelmäßig Kunststoffbecher zum Einsatz, welche einen erheblichen Platzbedarf haben. Insbesondere im mobilen Einsatz sind platzsparende Sets für den Nachweis von Analyten in einer vom menschlichen Organismus stammenden Analysen- oder Flüssigkeitsprobe erforderlich.

In der DE 20 2016 104 645 U1 ist eine Vorrichtung zum visuellen Nachweis von Analyten in einer flüssigen Probe beschrieben, die für den mobilen Einsatz geeignet ist und die nur wenig Platz beansprucht. Hierzu umfasst die Vorrichtung eine Trägerplatte mit Teststreifenaufnahmen, in denen jeweils ein mit einem Probeaufnahmeabschnitt und ein Testabschnitt versehener Teststreifen angeordnet ist, und die über eine nicht lösbar mit der Trägerplatte verbundene Deckschicht verschlossen ist, die im Bereich der Testabschnitt der Teststreifen der Teststreifenaufnahmen ein Kontrollfenster aufweist. Dabei ist die Trägerplatte aus Pappe hergestellt, wobei die Folie aus verrottbarem Material gebildet ist, wodurch die so gestaltete Kassette nicht nur äußerst platzsparend ausgebildet werden kann, sondern darüber hinaus auch umweltschonend zu entsorgen ist. Zur Aufnahme einer Urinprobe zur Aufgabe auf eine solche Kassette sind jedoch nach wie vor voluminöse Laborbecher im Einsatz, welche erheblich zu dem Platzbedarf der mitzuführen Sets beitragen. In der WO 2017/013446 A1 ist ein aus flächigen Zuschnitten herstellbares Flüssigkeitsbehältnis in verschiedenen Ausgestaltungen beschrieben.

Hier will die Erfindung Abhilfe schaffen. Der Erfindung liegt die Aufgabe zu Grunde, einen Laborbecher, insbesondere Urinbecher für den mobilen Einsatz bereitzustellen, der nur einen geringen Stauraum benötigt und der darüber hinaus umweltschonend zu entsorgen ist. Gemäß der Erfindung wird diese Aufgabe durch einen Laborbecher mit den Merkmalen des Patentanspruchs 1 gelöst. Mit der Erfindung ist ein Laborbecher, insbesondere Urinbecher bereitgestellt, der nur einen geringen Platzbedarf hat und der darüber hinaus umweltschonend zu entsorgen ist. Dadurch, dass der Becherkörper aus zwei Flachmaterialzuschnitten besteht, die aufeinander liegend angeordnet an drei Seiten verklebt sind, wobei die beiden Flachmaterialzuschnitte gegenüberliegend mit zwei Außenknickfalzen sowie einer zwischen diesen angeordneten Mittelknickfalz versehen sind, ist der für den Laborbecher erforderliche Stauraum minimiert. Der Laborbecher wird erst kurz vor dem Einsatz über seine Knickfalze auseinander gefaltet, wodurch das Aufnahmevolumen für die Flüssigkeitsprobe bereitgestellt wird. Über die gegenüberliegend angeformten beiden Standflügel ist der so erstellte Laborbecher kippsicher abstellbar.

In Weiterbildung der Erfindung sind die Flachmaterialzuschnitte aus flüssigkeitsdicht beschichteter Kartonage hergestellt. Hierdurch ist eine umweltgerechte Entsorgung ermöglicht. Dabei kommen bei der Herstellung vorzugsweise verrottbare Beschichtungen zum Einsatz.

In Ausgestaltung der Erfindung sind die beiden Flachmaterialzuschnitte identisch ausgebildet. Hierdurch ist eine kostengünstige Herstellung erzielt.

In weiterer Ausgestaltung der Erfindung sind die Flachmaterialzuschnitte an zwei gegenüberliegenden Seiten an den der Mittelknickfalz entgegengesetzten Seiten der Außenknickfalz flächig verklebt, wodurch die zwei gegenüberliegend angeordneten Flügel gebildet sind. Hierdurch ist die Herstellung weiter vereinfacht; eine separate Herstellung von Flügeln mit nachfolgender Befestigung entfällt.

In Weiterbildung der Erfindung sind die durch die Außenknickfalze gebildeten, an den Becherkörper angrenzenden Kanten der Flügel in Richtung der Becheröffnung zu einander winklig nach außen verlaufend angeordnet. Hierdurch ist eine gute Stabilität des Becherkörpers bewirkt. Dabei weist die Öffnung des Becherkörpers bevorzugt eine rautenförmige Kontur auf.

In Ausgestaltung der Erfindung ist der Laborbecher zu den Mittelknickfalzen symmetrisch ausgebildet. Hierdurch ist die Stabilität des Laborbechers verbessert.

In weiterer Ausgestaltung der Erfindung ist der Laborbecher an einem Flügel über eine Perforationslinie mit einer Kartonkassette zum visuellen Nachweis von Analyten in einer flüssigen Probe verbunden, die aus zwei entlang ihrer Längsachse schwenkbar miteinander verbundenen Trägerplatten gebildet ist, auf denen jeweils eine Zwischenschicht aufgebracht ist, die zwei Teststreifen aufnimmt und die mit einer Deckschicht versehen ist. Hierdurch ist ein einteiliges Set für den mobilen Einsatz bereitgestellt, dass äußerst platzsparend ist und sowohl eine Kartusche zum Nachweis von Analyten in einer flüssigen Probe, als auch den hierzu erforderlichen Laborbecher zur Aufnahme der flüssigen Probe, insbesondere einer Urinprobe bereitstellt. Eine solche, insbesondere aus Kartonage bzw. Wellpappe hergestellte Kartonkassette ist beispielsweise in der DE 20 2016 104 645 U1 beschrieben. Der hierzu hergestellte Kartonagenzuschnitt ist einteilig um die für den Laborbecher erforderlichen Flachmaterialzuschnitte ergänzt, die zum einfachen ablösen über eine Perforationslinie mit der Kartonagenkassette verbunden sind.

In Weiterbildung der Erfindung weist die über eine Perforationslinie mit dem Laborbecher verbundene Kartonkassette wenigstens eine Probenträgeraufnahme auf, die einen "dried spot"-Probenträger (DS-Probenträger), insbesondere einen "dried urine spot"-Probenträger (DUS-Probenträger) aufnimmt. Hierdurch können auf der Kartonkassette selbst einige wenige Tropfen einer Probenflüssigkeit bevorratet werden. Die definierten eingetrockneten Flüssigkeitsrückstände können sodann im Falle von Urin als sogenannte "dried urine spots" (DUS) gemeinsam mit den Teststreifen zur Bestätigungsanalyse gegeben werden. Der zuvor von der Kartonagenkassette getrennte Laborbecher kann direkt nach Gebrauch umweltgerecht entsorgt werden.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:
- Figur 1: die schematische Darstellung eines Laborbechers;
- Figur 2: die Darstellung des Laborbechers aus Figur 1 im zusammengefalteten Zustand und
- Figur 3: die schematische Darstellung eines Laborbechers in zusammengefalteten Zustand mit angeordneter Kartonkassette zum visuellen Nachweis einer vom menschlichen Organismus stammenden Analyse- oder Flüssigkeitsprobe.

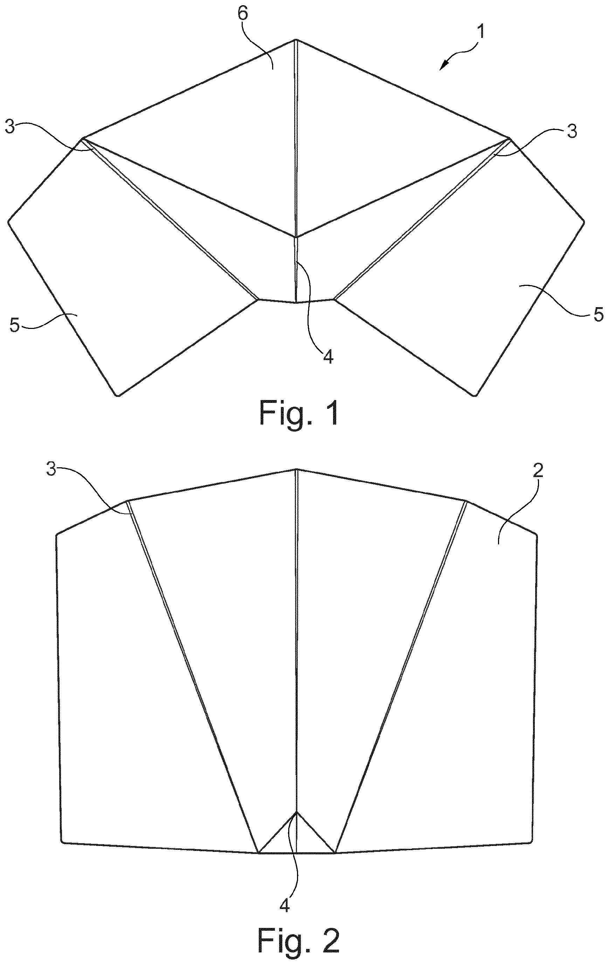

Der als Ausführungsbeispiel gewählte Laborbecher 1 besteht im Wesentlichen aus zwei identisch ausgebildeten Flachmaterialzuschnitten 2, die aufeinanderliegend angeordnet an drei Seiten miteinander verklebt sind, wobei die beiden Flachmaterialzuschnitte 2 gegenüberliegend mit zwei Außenknickfalzen 3 sowie einer zwischen diesen angeordneten Mittelknickfalz 4 versehen sind. Dabei sind die Flachmaterialzuschnitte 2 aus flüssigkeitsdicht beschichteter Kartonage hergestellt und an zwei gegenüberliegenden Seiten an den der Mittelknickfalz 4 entgegengesetzten Seiten der Außenknickfalze 3 flächig verklebt, wodurch zwei gegenüberliegend angeordnete Flügel 5 gebildet sind. Die durch die Außenknickfalze 3 gebildeten, an den Becherkörper 6 angrenzenden Kanten der Flügel 5 sind in Richtung der Becheröffnung zueinander winklig nach außen verlaufend angeordnet (vgl. Figur 1). Dabei weist die Öffnung des Becherkörpers im ausgefalteten Zustand eine trapezförmige Kontur auf. Der so ausgebildete Laborbecher ist zu den Mittelknickfalzen 4 symmetrisch ausgebildet.

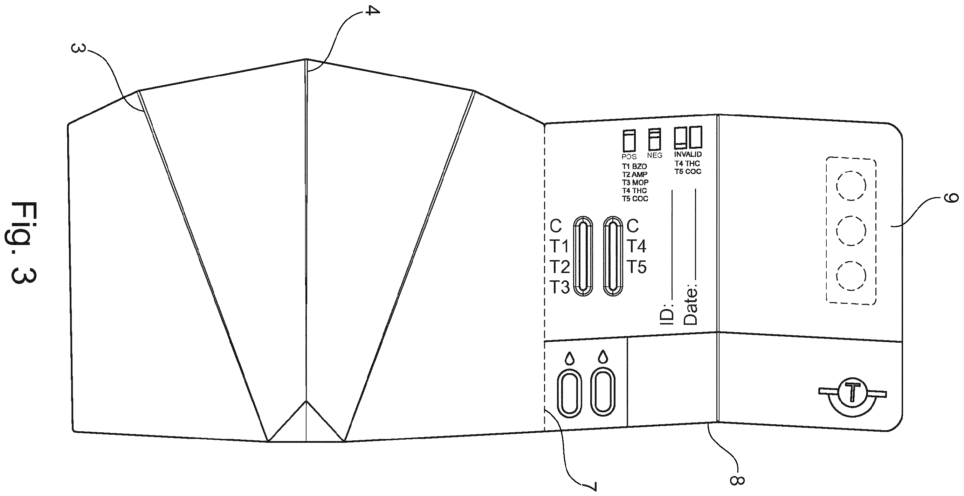

In Figur 3 ist der Laborbecher 1 über eine Perforationslinie 7 mit einer Kartonkassette 8 verbunden. Die Kartonkassette 8, deren Flachmaterialzuschnitte Bestandteil der Flachmaterialzuschnitte 2 des um die Kartonkassette ergänzten Laborbechers 1 sind, ist im Ausführungsbeispiel entsprechend der DE 20 2016 104 645 U1 ausgebildet. Der so gestaltete Laborbecher mit angeordneter Kartonagenkassette ist so in einem Zuge aus zwei identischen im Herstellungsprozess miteinander zu verklebenden Flachmaterialzuschnitten kostengünstig herstellbar sowie im Nachgang äußerst platzsparend zu verstauen.

In der vorzugsweisen Weiterbildung des in Figur 3 wiedergegebenen Laborbechers ist die Kartonkassette 8 zusätzlich mit eine Probenträger 9 versehen, die einen "dried urine spot"-Probenträger aufnimmt, wo nach der Entnahme mittels des Laborbechers einige Urintropfen archiviert werden können.

## Patentansprüche

1. Laborbecher (1), insbesondere Urinbecher, mit einem Becherkörper (6), an den gegenüberliegend zwei Standflügel (5) angeformt sind, bestehend aus zwei Flachmaterialzuschnitten (2), die aufeinanderliegend angeordnet an drei Seiten verklebt sind, wobei die beiden Flachmaterialzuschnitte (2) gegenüberliegend mit zwei Außenknickfalzen (3) sowie einer zwischen diesen angeordneten Mittelknickfalz (4) versehen sind, **dadurch gekennzeichnet, dass** die Flachmaterialzuschnitte(2) an zwei gegenüberliegenden Seiten an den der Mittelknickfalz (4) entgegengesetzten Seiten der Außenknickfalze (3) flächig verklebt sind, wodurch die zwei gegenüberliegend angeordneten Flügel (5) gebildet sind.

2. Laborbecher nach Anspruch 1, **dadurch gekennzeichnet, dass** die Flachmaterialzuschnitte (2) aus flüssigkeitsdicht beschichteter Kartonage hergestellt sind.

3. Laborbecher nach Anspruch 1 oder 2, **dadurch gekennzeichnet, dass** die beiden Flachmaterialzuschnitte (2) identisch ausgebildet sind.

4. Laborbecher nach einem der vorgenannten Ansprüche, **dadurch gekennzeichnet, dass** die durch die Außenknickfalze (3) gebildeten an den Becherkörper (6) angrenzenden Kanten der Flügel (5) in Richtung der Becheröffnung zueinander winklig nach außen verlaufend angeordnet sind.

5. Laborbecher nach einem der vorgenannten Ansprüche, **dadurch gekennzeichnet, dass** die Öffnung des Becherkörpers (6) eine rautenförmige Kontur aufweist.

6. Laborbecher nach einem der vorgenannten Ansprüche, **dadurch gekennzeichnet, dass** dieser zu den Mittelknickfalzen (4) symmetrisch ausgebildet ist.

7. Laborbecher nach einem der Ansprüche 2 bis 6, **dadurch gekennzeichnet, dass** an einem Flügel (5) eine Kartonkassette (8) zum visuellen Nachweis einer vom menschlichen Organismus stammenden Analyse-oder Flüssigkeitsprobe über eine Perforationslinie (7) mit diesem verbunden ist, die zwei entlang ihrer Längsachse schwenkbar miteinander verbundenen Trägerplatten aufweist, auf denen jeweils eine Zwischenschicht aufgebracht ist, die Teststreifen aufnimmt und die mit einer Deckschicht versehen ist.

8. Laborbecher nach Anspruch 7, **dadurch gekennzeichnet, dass** die Kartonkassette wenigstens eine Probenträgeraufnahme aufweist, die einen "dried spot"-Probenträger (DS-Probenträger), insbesondere einen "dried urine spot" - Probenträger (DUS - Probenträger) (9) aufnimmt.

## Claims

1. Laboratory cup (1), in particular a urine cup, having a cup body (6), on which two supporting wings (5) are oppositely formed consisting of two flat material blanks (2), which are arranged on top of each other and adhesively bonded on three sides, wherein the two flat material blanks (2) are oppositely provided with two outer folds (3) as well as a central fold (4) arranged between them, **characterised in that** the flat material blanks (2) are two-dimensionally bonded on two opposite sides on the sides of the outer fold (3) opposing the central fold (4), whereby the two oppositely arranged wings (5) are formed.

2. Laboratory cup according to claim 1, **characterised in that** the flat material blanks (2) are made of cardboard coated to be liquid-tight.

3. Laboratory cup according to claim 1 or 2, **characterised in that** the two flat material blanks (2) are of identical design.

4. Laboratory cup according to one of the previous claims, **characterised in that** the edges of the wings (5) formed by the outer folds (3) adjoining the cup body (6) are arranged at an angle with respect to one another extending outwardly in the direction of the cup opening.

5. Laboratory cup according to one of the previous claims, **characterised in that** the opening of the cup body (6) has a rhombic contour.

6. Laboratory cup according to one of the previous claims, **characterised in that** it is symmetrical with respect to the central folds (4).

7. Laboratory cup according to one of claims 2 to 6, **characterised in that** on one wing (5) a cardboard cassette (8) for the visual detection of an analysis or liquid sample derived from the human organism is connected therewith via a perforation line (7), which has two support plates pivotably connected to one another along its longitudinal axis, on which an intermediate layer is respectively applied, which receives test strips and which is provided with a cover layer.

8. Laboratory cup according to claim 7, **characterised in that** the cardboard cassette has at least one sample carrier receptacle, which receives a "dried spot" sample carrier (DS sample carrier), in particular a "dried urine spot" sample carrier (DUS sample carrier) (9).

## Revendications

1. Bécher de laboratoire (1), notamment bécher à urine, comprenant un corps (6) de bécher contre lequel ont été modelés deux volets verticaux (5) se faisant face, composé de deux découpes (2) de matériau plat au format, disposées superposées et collées sur trois côtés, sachant que les deux découpes (2) de matériau plat au format sont munies, se faisant face, de deux plis extérieurs (3) ainsi que d'un pli médian (4) disposé entre les deux, **caractérisé en ce que** les découpes (2) de matériau plat au format sont collées sur une certaine surface, sur deux côtés se faisant face, sur les côtés des plis extérieurs (3) opposés au pli médian (4), ce qui forme les deux volets (5) disposés se faisant face.

2. Bécher de laboratoire selon la revendication 1, **caractérisé en ce que** les découpes (2) de matériau plat au format sont fabriquées à partir d'un carton à revêtement étanche aux liquides.

3. Bécher de laboratoire selon la revendication 1 ou 2, **caractérisé en ce que** les deux découpes (2) de matériau plat au format dont configurées identiques.

4. Bécher de laboratoire selon l'une des revendications précédentes, **caractérisé en ce que** les bords des volets (5), limitrophes du corps (6) du bécher et formés par les plis extérieurs (3) sont, l'un par rapport à l'autre, disposés vers l'extérieur en formant un angle en direction de l'ouverture du bêcher.

5. Bécher de laboratoire selon l'une des revendications précédentes, **caractérisé en ce que** l'ouverture du corps (6) de bécher présente un contour losangé.

6. Bécher de laboratoire selon l'une des revendications précédentes, **caractérisé en ce que** ce dernier est configuré symétrique aux plis médians (4).

7. Bécher de laboratoire selon l'une des revendications 2 à 6, **caractérisé en ce que** contre un volet (5) une cassette (8) en carton est reliée avec lui via une ligne de perforation (7) pour livrer la preuve visuelle de présence d'un échantillon d'analyse ou de liquide provenant de l'organisme humain, ligne de perforation qui présente deux plaques support reliées entre elles de manière pivotante le long de son axe longitudinal, plaques sur lesquelles a été appliquée respectivement une couche intermédiaire qui reçoit des bandelettes réactives et qui est munie d'une couche de couverture.

8. Bécher de laboratoire selon la revendication 7, **caractérisé en ce que** la cassette en carton présente au moins un logement support d'échantillon recevant un support d'échantillon «dried spot» (support d'échantillon DS), en particulier un support d'échantillon «dried urine spot» (support d'échantillon DUS) (9).
